(19) **Europäisches Patentamt**

**European Patent Office**

**Office européen des brevets**

(11) Veröffentlichungsnummer: **0 301 246 B1**

(12) **EUROPÄISCHE PATENTSCHRIFT**

(45) Veröffentlichungstag der Patentschrift: **05.02.92**

(21) Anmeldenummer: **88110181.0**

(22) Anmeldetag: **25.06.88**

Teilanmeldung 91103352.0 eingereicht am 25/06/88.

(51) Int. Cl.⁵: **A61M 5/14**, A61M 5/32

(54) **Kanüle.**

(30) Priorität: **10.07.87 DE 3722800**

(43) Veröffentlichungstag der Anmeldung:
**01.02.89 Patentblatt 89/05**

(45) Bekanntmachung des Hinweises auf die
Patenterteilung:
**05.02.92 Patentblatt 92/06**

(84) Benannte Vertragsstaaten:
**AT BE CH DE ES FR GB IT LI LU NL SE**

(56) Entgegenhaltungen:
**US-A- 2 409 979**
**US-A- 2 697 438**
**US-A- 2 746 454**

(73) Patentinhaber: **B. Braun Melsungen AG
Carl-Braun Strasse
W-3508 Melsungen(DE)**

(72) Erfinder: **Haindl, Hans, Dr.
Forstgarten 22
W-3508 Melsungen(DE)**

(74) Vertreter: **Selting, Günther, Dipl.-Ing. et al
Patentanwälte von Kreisler, Selting, Werner
Deichmannhaus am Hauptbahnhof
W-5000 Köln 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung bezieht sich auf eine Kanüle mit einem starren Kanülenrohr, dessen eine Wandseite am vorderen Ende des Kanülenrohres eine gegen die gegenüberliegende axiale Wandseite gerichtete gekrümmte Abbiegung aufweist, in deren Verlauf in der axialen Wandseite eine Lumenöffnung ausgebildet ist, die zur Kanülenrohrlängsachse etwa parallel verläuft und der ein vorderer Einstechteil und eine im Verlauf der Innenfläche der axialen Wandseite liegende hintere innere Schneide zugeordnet ist.

Eine solche Kanüle ist aus US-A-2 746 454 bekannt. Dabei handelt es sich um Nadeln, deren Einführung in die Vene ohne Verletzungsgefahr der gegenüberliegenden Gefäßwand dadurch erleichtert werden soll, daß der vordere Einstechteil der Kanüle Hakenform erhält, wobei die der verhältnismäßig kurzen Lumenöffnung gegenüberliegende stark gekrümmte Rückenfläche als Gleitfläche wirksam ist. Die vordere runde Kante des Einstechteils ist quer zur Längsachse des Kanülenrohres zur Seite gerichtet und liegt in der Flucht der Außenfläche des Kanülenrohres. Sie ist frei von einem flachen Anschliff, während der hintere Rand der Lumenöffnung zur Bildung einer inneren Schneide flach angeschliffen ist. Eine solche Kanüle wird zur Ausnutzung ihrer gebogenen Gleitfläche auf einer gekrümmten Bahn in ein Blutgefäß eingeführt, deren Verlauf etwa dem Radius der Krümmung der Gleitfläche des Einstechteiles entspricht. Diese durch die hakenförmige Biegung erzwungene winklige Anstellung der Kanüle bei der Punktion führt dazu, daß die scharfe hintere Schneide der Lumenöffnung in diese hineindrängendes Material der zu durchstechenden Fläche ausstanzt, wodurch das Punktionsloch vergrößert wird. Dies ist bei Gewebepunktionen wegen der Traumatisierung nachteilig. Unerwünscht ist der Ausstanzeffekt jedoch auch, wenn die Kanüle zum Nachfüllen von Medikamenten-Applikationsvorrichtungen mit einer implantierten Kapsel verwendet wird, die einen Hohlraum zur Aufnahme des Medikamentes aufweist, an den ein Katheter angeschlossen ist. Das freie Ende des Katheters ist zu einer Infusionsstelle geführt und leitet dieser Medikamente zu. Die nach außen (zur Haut des Patienten hin) gerichtete Wand der implantierten Kapsel besteht aus einer perforierbaren Elastomer-Membran, die mit der Kanüle durchstochen wird, um Medikament in den Hohlraum der Kapsel nachzufüllen.

Das wiederholte Einstechen einer Kanüle in die Membran der Kapsel verursacht bisher Undichtigkeiten der Membran dadurch, daß mit der Schneide des Schliffauges Elastomermaterial ausgestanzt wird, so daß Löcher entstehen, die sich nicht mehr von selbst schließen. Nach einer verhältnismäßig geringen Zahl von Einstichen beginnt die Kapsel zu lecken, was den Anwender zum Auswechseln wenigstens der Kapsel der Applikationsvorrichtung zwingen kann. Außerdem führt die Mitnahme von ausgestanzten Materialteilchen in das Medikamentensystem zu einer Kontamination des Medikaments.

Als Nachfüllkanülen verwendet man bisher sogenannte Huber-Kanülen. Bei der derzeit benutzten Ausführungsform ist das ganze Kanülenrohr am vorderen Ende schräg zur Seite abgeknickt und mit einer im wesentlichen parallel zur Längsachse des Kanülenrohres verlaufenden, ringsum angeschliffenen Lumenöffnung am vorderen Ende des abgeknickten Kanülenrohrabschnittes versehen.

Die Lumenöffnung befindet sich dabei weit außerhalb der Kontur des geraden Kanülenrohres. Diese Tatsache bewirkt selbst bei ordnungsgemäßem senkrechtem Ansetzen der Huber-Kanüle Ausstanzungen von Materialpartikeln, weil der abgewinkelte vordere Kanülenrohrabschnitt einseitig keilartig durch die Fläche dringt und die dem Keil abgewandte hintere Schneide des Anschliffes der Lumenöffnung seitlich gegen das zu durchdringende Material gedrückt wird und in die Öffnung eingedrungenes Material abschert. Bei unsachgemäßem schrägen Einstechen der Kanülenspitze in die zu punktierende Fläche ergibt sich ein vermehrtes Eindringen von Wandmaterial in die Lumenöffnung mit der Folge, daß noch größere Materialpartien mit der exponierten Schneide des hinteren Schliffauges der Lumenöffnung abgeschert werden.

Bei einer anderen Ausführungsform der Huber-Kanüle mit seitwärts gerichteter Lumenöffnung (US-A-2 409 979) ist die eine Wandseite des Kanülenrohres ohne Krümmung schräg in Richtung der gegenüberliegenden axialen Wandseite abgeknickt. Die die Lumenöffnung umgebende Umrandung ist tangential zur Kanülenrohraußenfläche eben geschliffen und am hinteren Rand mit einer von außen nach innen abfallenden, d.h. äußeren, Schneide versehen. Der Einstechteil, dessen Spitze in der Flucht des Außenumfanges der axialen Wandseite liegt, ist auf der Umrandung der Lumenöffnung mit Facettenschliff versehen. Es hat sich gezeigt, daß auch die abgeknickte Schrägfläche des Kanülenrohres einen einseitigen Keileffekt bei der Punktion einer künstlichen oder natürlichen Fläche bewirkt, der die Ausbildung eines nicht aufgeweiteten, scharfen Schnittes beeinträchtigt und der beim Vorschub der Kanüle Material in die Lumenöffnung drängt, das an der hinteren Schneide abgehobelt und durch Quetschung beschädigt wird. Das Problem beträchtlicher Wandausstanzungen mit der hinteren Schneide eines Schliffauges ergibt sich auch bei einer anderen bekannten Kanüle (DE-OS 30 13 384), die in ihrem vorderen Bereich eine angeschliffene schräge Lumenöffnung aufweist, von deren vorderem Ende eine Spitze abgewinkelt ist. Das äußere Ende der abgewinkelten Spitze liegt hierbei etwa auf der Mittelachse des Kanülenrohres, während die hintere Schneide des Schliffauges sich am hinteren Ende der Lumenöffnung innerhalb der Kontur des Kanülenrohres befindet. Die durch die gegenseitige Ausrichtung der Teile des vorderen Kanülenrohrendes bedingte Führung der Spitze auf einer S-förmigen Kurvenbahn beim Einstich in eine Punktionsfläche führt zu erheblichem Stanzeffekt, der bei der Punktion von künstlichen und natürlichen Wandungen (Membran oder Körpergewebe) gleichermaßen unerwünscht ist.

Der Erfindung liegt die Aufgabe zugrunde, die eingangs erwähnte Kanüle so zu verbessern, daß bei der Punktion einer natürlichen oder künstlichen Wandung Ausstanzungen vermieden werden, um bei Anwendung der Kanüle, z.B. als Spinalkanüle, Gewebeschäden zu unterbinden und bei Anwendung als Nachfüllkanüle einen leckfreien Zustand der implantierten Kapsel einer Medikamenten-Applikationsvorrichtung nach mehreren hundert Punktionen zu gewährleisten.

Diese Aufgabe wird erfindungsgemäß dadurch gelöst, daß der Einstechteil eine lanzettförmige Spitze mit Facettenschliff aufweist, die in dem Bereich zwischen zwei gedachten parallelen Linien liegt, welche die Innenfläche und die Außenfläche der axialen Wandseite über die hintere Schneide der langgestreckten Lumenöffnung hinaus nach vorne verlängern.

Mit der erfindungsgemäßen Kanüle ergibt sich ein verringerter Stanzeffekt bei der Punktion künstlicher oder natürlicher Wandungen, weil die Position der lanzettförmigen, durch Facettenschliff scharfen Spitze des Einstechteils in der radialen Zone der axialen Wand gemeinsam mit der gekrümmten Abbiegung der Rückseite des Kanülenrohres bewirkt, daß ohne schädliche Keilwirkung in der zu durchdringenden Wand ein scharf getrennter Schlitz erzeugt wird, der von dem der Lumenöffnung gegenüberliegenden gebogenen Rücken bis auf den Außendurchmesser des geraden Kanülenrohres konzentrisch aufgeweitet wird und der sich nach Herausziehen der Kanüle jedesmal dicht verschließt. Die kombinierten Merkmale erleichtern die für die Unterbindung der Materialabnahme günstige senkrechte Einstichrichtung der Kanüle, und der Einstich bleibt klein, weil die seitliche Versetzung der Spitze des Einstechteils zur Längsmittelachse durch die Krümmung der Abbiegung so ausgeglichen wird, daß sich im Effekt der Einstich einer zentralen Kegelspitze ergibt. Der ohne Seitenabweichungen präzise koaxial erfolgende Durchtritt des Einstechteiles der Kanüle durch den Trennschlitz in lebendem Gewebe verhindert Gewebetraumatisierung und beschleunigt den Heilungsprozeß. Die messerartige Durchtrennung der Materialschicht ohne Materialabnahme läßt bis zu mehreren tausend Punktionen der elastischen Membran der implantierten Kapsel einer Medikamenten-Applikationsvorrichtung zu, ohne daß Lecks entstehen. Die Kapsel kann daher solange implantiert bleiben, wie die jeweilige Therapie verlangt.

Der Patient wird durch das Unterbleiben des mehrmaligen subkutanen Einpflanzens einer Kapsel und durch die Verhinderung des Einschleppens von ausgestanzten Partikeln in seinen Körper geschont. Die Therapie wird auf diese Weise zuverlässiger.

Die angeschliffene innere hintere Schneide der Lumenöffnung verläuft in der Flucht der Innenfläche des Kanülenrohres, und die Spitze des Einstechteils liegt entweder in der Flucht der hinteren Schneide oder an irgendeiner Stelle zwischen der gedachten Schneiden-Verlängerung und der Flucht des Außenumfanges des Kanülenrohres.

In vorteilhafter Ausgestaltung der Erfindung ist vorgesehen, daß der Facettenschliff in den ebenen Rand der Lumenöffnung eingearbeitet ist. Zu beiden Seiten der Längsmittelebene der Lumenöffnung des Kanülenrohres sind in zwei Ebenen angeordnete Schliffflächen eines Facettenschliffs ausgebildet, so daß eine scharfe und spitze Spitze des Einstechteils entsteht. Der Facettenschliff kann an der Lumenöffnung enden. Alternativ kann der Facettenschliff mit Abstand vor der Lumenöffnung enden. In diesem Falle ist der Facettenschliff auf ein kurzes Stück der Spitze des Einstechteiles beschränkt und der die Lumenöffnung umgebende ebene Rand hat die Form eines unfacettierten geschlossenen Anschliffes.

Zur Bildung der lanzettförmigen Spitze des Einstechteiles kann bei einer weiteren Ausführungsform der Erfindung der Einstechteil auf der Rückenseite einen Facettenschliff aufweisen. In diesem Falle ist der gesamte Rand der Lumenöffnung in ihrer Ebene von einem glatten ebenen Randanschliff geschlossen umgeben. Diese Ausführungsform ist herstellungsmäßig günstig.

Die angeschliffene hintere Schneide der Lumenöffnung ist vorzugsweise einwärts gerundet. Die Rundung trägt zusätzlich zur Vermeidung von Materialausstanzungen bei, weil sie beim Durchtritt der Kanüle durch die punktierte Fläche das Material wegdrückt und keinesfalls abschabt.

Der Biegewinkel der gekrümmten Abbiegung der einen Wandseite des Kanülenrohres liegt vorteilhafterweise in einem Bereich von $13° \pm 3°$, vorzugsweise bei $14°$. Als Biegewinkel wird der Winkel zwischen der Längsmittelachse des Kanülenrohres und der an die der Lumenöffnung gegenüberliegende gebogene Rückenseite des vorderen Endes des Kanülenrohres angelegten Tangente bezeichnet.

In der Zeichnung sind Ausführungsbeispiele der Erfindung schematisch dargestellt. Es zeigt:

Fig. 1 eine Seitenansicht einer Kanüle,

Fig. 2 eine um 90° gedrehte Draufsicht auf die Kanüle nach Figur 1,

Fig. 3 einen Längsschnitt durch den Einstechteil der Kanüle nach Figur 2 längs der Linie III-III in vergrößertem Maßstab,

Fig. 4 eine Seitenansicht einer zweiten Ausführungsform einer Kanüle,

Fig. 5 eine um 90° gedrehte Draufsicht auf die Kanüle nach Figur 4,

Fig. 6 eine Seitenansicht einer dritten Ausführungsform einer Kanüle und

Fig. 7 eine um 90° gedrehte Draufsicht auf die Kanüle nach Figur 6.

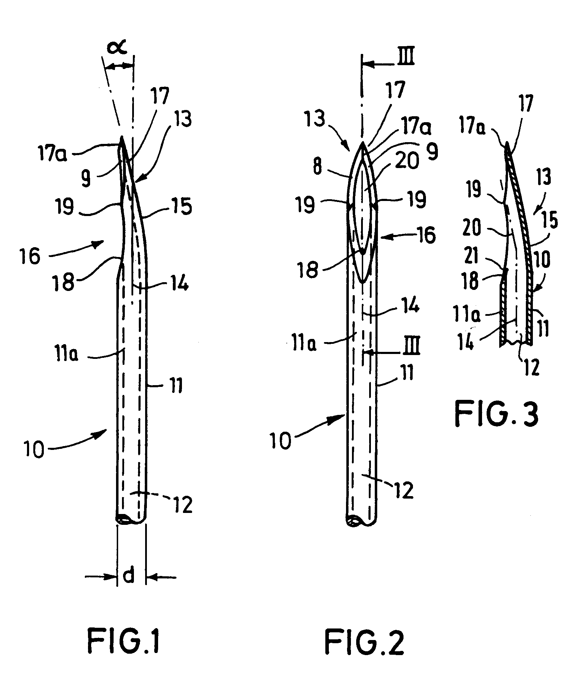

Die Kanüle 10 nach Figuren 1 und 2 besteht aus einem zylindrischen starren Kanülenrohr 11 aus Metall, dessen hinteres Ende z.B. mit einer Injektionsspritze oder einem Schlauch und dergleichen zum Anschluß eines Flüssigkeits-überleitungssystems verbindbar ist. Das Kanülenrohr 11 ist über den größten Teil seiner Länge gerade. Sein Außendurchmesser d beträgt bei dem gezeigten Beispiel 0,7 mm. Der Durchgangskanal 12 kann einen Durchmesser von 0,4 mm aufweisen. Das vordere Ende des Kanülenrohres 11 ist zur Bildung eines Einstechteiles 13 auf einer Wandseite gekrümmt schräg zur Seite abgebogen, während die gegenüberliegende Wandseite 11a axial stehenbleibt, so daß eine ovale Lumenöffnung 20 entsteht, die zur Kanülenrohr-Längsachse 14 etwa parallel verläuft und deren Rand 16 ringsum angeschliffen ist. Die Abbiegung erfolgt im Verlauf der Lumenöffnung 20. Der Rand 16 ist auf der vorderen Hälfte als Facettenschliff mit zwei in zwei Ebenen symmetrisch angeordneten Schliffflächen 8,9 so gestaltet, daß eine axial gerichtete scharfe Spitze 17 am vorderen Schliffauge entsteht. Der Anschliff der hinteren Hälfte des Randes 16 bildet ein hinteres Schliffauge mit einer runden Schneide 18, die im Verlauf der Innenfläche der axialen Wandseite 11a liegt. Die scharfe Kante der hinteren Schneide 18 ist bei 21 durch Glasperlenstrahlen verrundet (Fig. 3). Die beiden Schliffaugen sind durch zwei schräge Grate 19 voneinander getrennt und die Anschliffpartie des hinteren Schliffauges zwischen den Graten 19 und der Schneide 18 verläuft konkav (Fig. 1). Auf diese Weise bleibt die Schneide 18 des hinteren Schliffauges biegungsfrei so stehen wie bei dem Facettenschliff einer nicht abgewinkelten geraden Injektionskanüle, d.h. sie steht nicht über die Kontur des Kanülenrohres 11 nach außen vor, sondern liegt um das Maß der Wandstärke des Kanülenrohres 11 innerhalb seiner Kontur. Die Spitze 17, die eine schräge axiale scharfe Einstechkante 17a aufweist, von der nach beiden Seiten symmetrisch die Schliffflächen 8, 9 des Facettenschliffes zu den Graten 19 verlaufen, liegt im wesentlichen in der Flucht zwischen zwei gedachten parallelen Linien, die von der Innenfläche und der Außenfläche der axialen Wandseite 11a des Kanülenrohres 11 nach vorne ausgehen. Die Einstech- und Schneidteile des Kanülenrohres 11 liegen innerhalb der Kontur des Kanülenrohres 11 im Bereich der Wandstärke der axialen Wandseite 11a, wodurch bei hervorragender Trenneigenschaft ein Ausstanzen von Partikeln aus der zu durchdringenden natürlichen oder künstlichen Materialschicht verhindert wird. Unter natürlicher Materialschicht wird Gewebe verstanden, das bei Anwendung der Kanüle 10 als Spinalkanüle durchdrungen wird. Eine künstliche Materialschicht wäre z.B. eine Elastomer-Membran bei einer implantierten Kapsel eines Medikamenten-Applikationssystems. Die Länge der Biegung der Rückenseite 15 des Einstechteiles 13 zwischen dem äußeren Punkt der Spitze 17 und der Schneide 18 der Lumenöffnung 20 beträgt bei dem gezeigten Beispiel 3,6 d ± 0,42 d. Sie kann innerhalb der beanspruchten Toleranz länger sein, sofern sie im Schliffverlauf verbleibt. Der Biegewinkel alpha zwischen der Längsmittelachse 14 des Kanülenrohres 11 und der an die gerundet gebogene Rückenseite 15 des Einstechteiles 13 des Kanülenrohres 11 angelegten Tangente beträgt vorzugsweise 13° ± 1°.

Die von dem angeschliffenen Rand 16 umgebene, zur Seite gerichtete Lumenöffnung 20 des Kanülenrohres 11 hat die Form einer langgestreckten Doppellanzette, deren beiden Längshälften zu der durch die Längsachse 14 des Kanülenrohres 11 verlaufenden Ebene symmetrisch sind und deren vordere Querhälfte den Facettenschliff 8,9 aufweist.

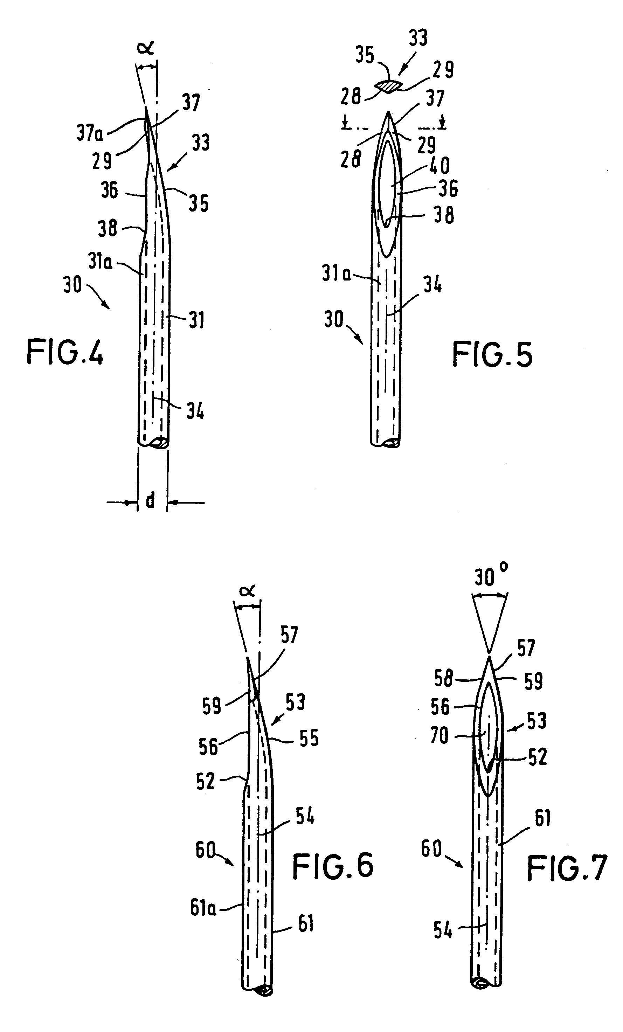

Bei dem Beispiel der Figuren 4 und 5 ist ein kreiszylindrisches starres Kanülenrohr 31 einer Kanüle 30 mit einem abgewandelten Einstechteil 33 versehen. In diesem Falle ist die zur Kanülenrohr-Längsachse 34 etwa parallele ovale Lumenöffnung 40 von einem Rand 36 umgeben, der ringsum ebenflächig angeschliffen ist. Der hintere Rand der Lumenöffnung 40 bildet eine scharfe innere Schneide 38, die im Verlauf der Innenseite der axialen Wand 31a liegt und - wie in Figur 3 gezeigt - einwärts gerundet sein kann. An den ebenflächigen Anschliff des Randes 36 schließt sich an der Spitze 37 ein Facettenschliff an, der aus zwei Schliffflächen 28, 29 zusammengesetzt ist, die in zwei Ebenen symmetrisch angeordnet sind und an einer axialen scharfen Einstechkante 37a der Spitze 37 des Einstechteils 33 zusammenstoßen. Die Spitze 37 mit der Einstechkante 37a liegt auch bei diesem Beispiel in dem Bereich zwischen zwei gedachten parallelen Linien, die die Innenfläche und die Außenfläche der axialen Wandseite 31a über die hintere Schneide 38 der Lumenöffnung 40 hinaus nach vorne verlängern. Diese Ausbildung sorgt dafür, daß praktisch keine Partikel aus der zu durchdringenden Materialschicht ausgestanzt werden. In Figur 5 ist ein Querschnitt des Einstechteiles 33 im Bereich des Facettenschliffes gezeigt.

Die gekrümmte Rückenseite 35, in deren Verlauf die gegenüberliegende Lumenöffnung 40 sich befindet, hat eine Länge von 3,6d ± 0,42d. Der Biegewinkel alpha kann auch bei diesem Beispiel 13° ± 1° betragen.

Gemäß Figuren 6 und 7 ist ein Kanülenrohr 61 einer Kanüle 60 am vorderen Ende unter Bildung einer gerundet gebogenen Rückenseite 55 mit einem Einstechteil 53 versehen, der eine seitwärts gerichtete Lumenöffnung 70 aufweist, die etwa parallel zur Längsachse 54 des Kanülenrohres 61 verläuft. Der Rand 56 der Lumenöffnung 70 ist ebenflächig angeschliffen und umgibt die Lumenöffnung 70 ringsum. Ihr hinterer Rand bildet eine innere Schneide 52. Eine Spitze 57 des Einstechteiles 53 ist zur Längsachse 54 des Kanülenrohres 61 seitlich versetzt und dadurch angeschärft, daß sie auf der Rückenseite 55, also gegenüber dem angeschliffenen Rand 56, einen als Hinterschliff bezeichneten Facettenschliff mit zwei Schliffflächen 58, 59 aufweist. Vorzugsweise sind die in zwei Ebenen verlaufenden Schliffflächen 58,59 des Facettenschliffes unter einem Winkel von 30° zueinander angestellt. Bei diesem Beispiel ist der Rand 56 der Lumenöffnung 70 von der Schneide 52 bis zur Spitze 57 gerade und ebenflächig. Die Anordnung der Spitze 57 in bezug auf die axiale Wandseite 61a des Kanülenrohres 61 entspricht derjenigen der vorangegangenen Beispiele.

**Patentansprüche**

1. Kanüle mit einem starren Kanülenrohr (11), dessen eine Wandseite am vorderen Ende des Kanülenrohres (11) eine gegen die gegenüberliegende axiale Wandseite (11a) gerichtete gekrümmte Abbiegung (15) aufweist, in deren Verlauf in der axialen Wandseite (11a) eine Lumenöffnung (16) ausgebildet ist, die zur Kanülenrohr-Längsachse (14) etwa parallel verläuft und der ein vorderer Einstechteil (13) und eine im Verlauf der Innenfläche der axialen Wandseite (11a) liegende hintere innere Schneide (18) zugeordnet ist, **dadurch gekennzeichnet,** daß der Einstechteil (13) eine lanzettförmige Spitze (17) mit Facettenschliff (8,9) aufweist, die in dem Bereich zwischen zwei gedachten parallelen Linien liegt, welche die Innenfläche und die Außenfläche der axialen Wandseite (11a) über die hintere Schneide (18) der langgestreckten Lumenöffnung (20) hinaus nach vorne verlängern.

2. Kanüle nach Anspruch 1, **dadurch gekennzeichnet,** daß der Facettenschliff (8,9) in den ebenen Rand (16) der Lumenöffnung (20) eingearbeitet ist.

3. Kanüle nach Anspruch 2, **dadurch gekennzeichnet,** daß der Facettenschliff (8,9) an der Lumenöffnung (20) endet.

4. Kanüle nach Anspruch 2, **dadurch gekennzeichnet,** daß der Facettenschliff (28,29) mit Abstand vor der Lumenöffnung (40) endet.

5. Kanüle nach Anspruch 1, **dadurch gekennzeichnet,** daß der Facettenschliff (58,59) als Hinterschliff auf der Rückenseite (55) des Einstechteils (53) ausgebildet ist.

6. Kanüle nach einem der Ansprüche 1 bis 5, **dadurch gekennzeichnet,** daß die hintere Schneide (18) der Lumenöffnung (20) einwärts gerundet ist.

7. Kanüle nach einem der Ansprüche 1 bis 6, **dadurch gekennzeichnet,** daß die gekrümmte Abbiegung (15) sich über einen Längenbereich von 2d bis 5d erstreckt, wobei d der äußere Kanülenrohrdurchmesser ist.

8. Kanüle nach einem der Ansprüche 1 bis 7, **dadurch gekennzeichnet,** daß der Biegewinkel (alpha) in einem Bereich von 13° ± 3° liegt und vorzugsweise 14° beträgt.

**Claims**

1. Cannula comprising a hollow rigid cannula tube (11) having one wall side at the front end of the cannula tube (11) provided with a curved bending (15) directed towards the opposite axial wall side (11a), a lumen opening (16) being formed in said axial wall side (11a) in the region of said curved bending (15), which lumen opening (16) extends substantially parallel to the longitudinal axis (14) of the cannula tube (11) and is provided with a front punctuating portion (13) and a rear inner cutting edge (18) arranged on the inner surface of said axial wall side (11a), **characterized in that** the punctuating portion (13) comprises a lancet-shaped tip (17) having a facet grinding (8, 9) and being arranged in the zone between two imaginary parallel lines which continue the inner surface and the outer surface of said axial wall side (11a) forwardly beyond the rear cutting edge (18) of the elongate lumen opening (20).

2. Cannula according to claim 1, characterized in that the facet grinding (8, 9) is worked into the flat edge (16) of the lumen opening (20).

3. Cannula according to claim 2, characterized in that the facet grinding (8, 9) ends at the lumen opening (20).

4. Cannula according to claim 2, characterized in that the facet grinding (28, 29) ends at a distance before said lumen opening (40).

5. Cannula according to claim 1, characterized in that the facet grinding (58, 59) is formed as a rear bevel on the back side (55) of said punctuating portion (53).

6. Cannula according to any one of claims 1 to 5, characterized in that the rear cutting edge (18) of said lumen opening (20) is rounded inwardly.

7. Cannula according to any one of claims 1 to 6, characterized in that the curved bending (15) extends over a lengthwise area of 2d to 5d wherein d is the outer diameter of the cannula tube.

8. Cannula according to any one of claims 1 to 7, characterized in that the bending angle ($\alpha$) lies in an area from $13^\circ \pm 3^\circ$ and preferably amounts to $14^\circ$.

**Revendications**

1. Canule comportant un tube de canule rigide (11) dont l'une des parois latérales à l'extrémité avant du tube de canule (11) présente un coude courbe (15) orienté en direction de la paroi latérale axiale opposée (11a), et sur l'étendue duquel est réalisée, dans la paroi latérale axiale (11a), une lumière (16) qui s'étend de manière sensiblement parallèle à l'axe longitudinal (14) du tube de canule et à laquelle est associée une partie avant à piquer (13) et une arête de coupe intérieure arrière (18) située sur l'étendue de la surface intérieure de la paroi latérale axiale (11a), caractérisée en ce que la partie à piquer (13) comporte une pointe (17) qui présente une forme de lancette avec un affûtage en facettes (8, 9), et qui est située dans la zone entre deux lignes parallèles fictives prolongeant vers l'avant au-delà de l'arête de coupe arrière (18) de la lumière allongée (20), la surface intérieure et la surface extérieure de la paroi latérale axiale (11a).

2. Canule selon la revendication 1, caractérisée en ce que l'affûtage en facettes (8, 9) est usiné dans la bordure plane (16) de la lumière (20).

3. Canule selon la revendication 2, caractérisée en ce que l'affûtage en facettes (8, 9) se termine au niveau de la lumière (20).

4. Canule selon la revendication 2, caractérisée en ce que l'affûtage en facettes (28, 29) se termine à distance de la lumière (40).

5. Canule selon la revendication 1, caractérisée en ce que l'affûtage en facettes (58, 59) est réalisé sous la forme d'un affûtage en dépouille sur la face arrière (55) de la partie à piquer (53).

6. Canule selon l'une des revendications 1 à 5, caractérisée en ce que l'arête de coupe arrière (18) de la lumière (20) est arrondie vers l'intérieur.

7. Canule selon l'une des revendications 1 à 6, caractérisée en ce que le coude courbe (15) s'étend sur une longueur allant de 2d à 5d, d étant le diamètre extérieur du tube de canule.

8. Canule selon l'une des revendications 1 à 7, caractérisée en ce que l'angle du coude ($\alpha$) se situe dans une plage de $13^\circ \pm 3^\circ$, et vaut de préférence $14^\circ$.

FIG.1

FIG.2

FIG.3

FIG.4

FIG.5

FIG.6

FIG.7